# EUROPEAN PATENT SPECIFICATION

(11) **EP 4 271 477 B1**
(45) Date of publication and mention of the grant of the patent: **02.12.2026**
(21) Application number: 21916076.9
(22) Date of filing: 30.12.2021
(51) Int. Cl.: A61K 31/47, A61K 45/06, A61P 11/00, A61P 11/06, A61P 31/14

(54) **MONTELUKAST DERIVATIVE FOR DUAL INHIBITION OF SARS-COV-2 VIRUS**
MONTELUKAST-DERIVATE ZUR DOPPELTEN HEMMUNG DES SARS-COV-2-VIRUS
DÉRIVÉS DE MONTÉLUKAST POUR L'INHIBITION DOUBLE DU VIRUS SARS-COV-2

(30) Priority: 30.12.2020 TR 202022556
(43) Date of publication of application: 08.11.2023
(73) Proprietor: Bahçesehir Üniversitesi, 34349 Besiktas/Istanbul (TR)
(72) Inventor: DURDAGI, Serdar, 34353 Besiktas/Istanbul (TR); AVSAR, Timucin, 34353 Besiktas/Istanbul (TR); GUZEL, Mustafa, 34810 Beykoz/Istanbul (TR); BALCIOGLU, Bertan Koray, Gebze/Kocaeli (TR); CETIN, Yuksel, Gebze/Kocaeli (TR); SERHATLI, Muge, Gebze/Kocaeli (TR); OZTURK, Hasan Umit, Gebze/Kocaeli (TR)
(74) Representative: Genç Ilhan, Oznur
(86) International application number: PCT/TR2021/051637
(87) International publication number: WO 2022/146399

(56) References cited:
- BOZEK ANDRZEJ ET AL: "Montelukast's ability to fight COVID-19 infection", JOURNAL OF ASTHMA, vol. 58, no. 10, 26 June 2020 (2020-06-26), US, pages 1348 - 1349, XP093182754, ISSN: 0277-0903, DOI: 10.1080/02770903.2020.1786112
- ABU-SALEH ABD AL-AZIZ ET AL: "Discovery of potent inhibitors for SARS-CoV-2's main protease by ligand-based/structure-based virtual screening, MD simulations, and binding energy calculations", PHYSICAL CHEMISTRY CHEMICAL PHYSICS, vol. 22, no. 40, 21 October 2020 (2020-10-21), pages 23099 - 23106, XP093182785, ISSN: 1463-9076, DOI: 10.1039/D0CP04326E
- DURDAGI SERDAR ET AL: "The neutralization effect of montelukast on SARS-CoV-2 is shown by multiscale in silico simulations and combined in?vitro studies", MOLECULAR THERAPY, vol. 30, no. 2, 19 October 2021 (2021-10-19), US, pages 963 - 974, XP093182774, ISSN: 1525-0016, DOI: 10.1016/j.ymthe.2021.10.014
- MAFFUCCI, I. ET AL.: "In silico drug repurposing for SARS-CoV-2 main proteinase and spike proteins", JOURNAL OF PROTEOME RESEARCH, vol. 19, no. 11, 2020, pages 4637 - 4648, XP055954029
- ALMERIE, M. Q. ET AL.: "The association between obesity and poor outcome after COVID-19 indicates a potential therapeutic role for montelukast", MEDICAL HYPOTHESES, vol. 143, 2020, pages 109883, XP086271912, DOI: 10.1016/j.mehy.2020.109883
- QIAO, Z. ET AL.: "Computational view toward the inhibition of SARS-CoV-2 spike glycoprotein and the 3CL protease", COMPUTATION, vol. 8, no. 2, 2020, pages 53, XP055839968, DOI: 10.3390/computation8020053
- SUCEVEANU, A. I. ET AL.: "Assertion for montelukast in the covid-19 pandemics", FARMACIA, vol. 68, no. 4, 2020, pages 579 - 585, XP55954040

## Description

### Field of the Invention

The present invention relates to the use of a montelukast derivative to inactivate the SARS-CoV-2 virus that causes COVID-19 disease. The present invention also relates to a montelukast derivative for use in the prevention, delay of progression, relief of symptoms and treatment of COVID-19 disease. The present invention also includes pharmaceutical formulations containing a montelukast derivative for use in the indication of COVID-19.

### Background Art

The new coronavirus, which emerged towards the end of 2019 and still continues today, causing an epidemic affecting more than 283 million people worldwide, has been called Severe Acute Respiratory Syndrome Coronavirus 2 (SARS-CoV-2) (World Health Organization, 2020).

Coronaviruses are enveloped, non-segmented positive-sense RNA viruses belonging to the family of Coronaviridae, and widely distributed in humans, other mammals and birds, causing respiratory, enteric, hepatic and neurological diseases. Of the seven types of coronaviruses that cause the disease, especially viruses 229E, OC43, NL63 and HKU1 are quite common. While said viruses cause cold-like symptoms, cases caused by SARS-CoV, MERS-CoV and SARSCoV-2 viruses may result in death.

In addition to the common cold symptoms such as fever, cough, and sore throat, persons infected with the SARS-CoV-2 virus have many clinical signs such as severe pneumonia, clot formation and endothelitis, fatigue, neurological and cardiac symptoms, kidney dysfunction, shortness of breath, respiratory distress, and muscle-joint pain. In some cases, death occurs because of multiple organ failure.

Studies on COVID-19 have demonstrated the relationship between COVID-19 and the most common chronic conditions such as diabetes, cardiovascular diseases, respiratory system diseases, immune system disorders. Asthma and chronic obstructive pulmonary disease (COPD) are among the most common respiratory diseases. Asthma is a chronic inflammatory airway condition, and there is substantial evidence in that patients with asthma are predisposed to viral infections.

All coronaviruses have specific genes in their protein-coding ORF1 downstream regions for viral replication, nucleocapsid and spike development. SARS-CoV-2 virus has four structural proteins which are nucleocapsid, envelope, membrane and spike. These four proteins play a vital role during viral infection. The glycoprotein spike structures located on the external surface of coronaviruses are responsible for the connection and entry of the virus to host cells. Like other coronaviruses, the S protein of SARS-CoV-2 mediates mechanisms such as receptor recognition, cell attachment, and fusion during viral infection. While the virus is in its natural environment, S protein of the coronavirus is inactive. During viral infection, target cell proteases activate the S protein by cleaving it into S1 and S2 subunits, which are required to activate the membrane fusion domain after viral entry into target cells. The S1 subunit includes the receptor binding domain (RBD), which binds directly to the peptidase domain (PD) angiotensin converting enzyme-2 (ACE2). S2 functions during membrane fusion.

In persons infected with the SARS-CoV-2 virus through the skin or mucous membranes, SARS-CoV-2 virus binds to the ACE-2 receptors in the cells with the Spike protein on its surface with high affinity, and moves into the cell. After the virus enters into the cytoplasm, it releases the genome. After the virus genome is released into the cytoplasm, the RNA of the virus combines with the RNA of the host cell, thus causing the initiation of infection. Considering the role of Spike protein in binding to the host cell, fusion and entry into the cell, it is clear that the Spike protein should be inactivated in the treatment of COVID-19.

On the other hand, for the life cycle of the SARS-CoV-2 virus, a chymotrypsin-like cysteine protease called main protease (which can also be called as 'Mpro', main protease, 3CL protease or 3CL-pro) is needed. Mpro is a vital enzyme involved in processes such as processing, assembly and replication of the virus, and the role of Mpro is vital for the virus to complete its life cycle. In view of the studies conducted on epidemic coronoviruses that emerged in the past years, Mpro is known to be one of the most important targets for antiviral drugs. Therefore, in order to inactivate the SARS-CoV-2 virus, inhibition of Mpro is among the important target structures in the treatment of COVID-19.

In order to quickly eliminate or reduce the negative effects of the COVID-19 disease on the whole world, the search for an effective treatment method has emerged. In order to inactivate the SARS-CoV-2 virus, it is demonstrated that the SARS-CoV-2 virus must be prevented from binding to the cell and replicating. Although comprehensive drug and vaccine studies are carried out for this purpose, in particular, an effective solution has not yet been found in the treatment of COVID-19 by drugs.

Today, it has become a preferred technique to use drugs that have been approved and available on the market for different indications. Developing new drugs from scratch is a long process and therefore it is impractical to tackle with the current global challenge. Many drugs have several protein targets, and some of the diseases involve overlapping molecular mechanisms. In this case, both the cost of the drug development process will decrease, and the time for the research and development and possible risks will be significantly reduced.

Active substances such as vitamin D2, remdesivir, hydroxychloroquine, dexamethasone, which are already approved in Europe and America, are some of the active substances recommended for the treatment of COVID-19. Also, Pfizer and Merck companies received emergency approval for the drugs Paxlovid and Molnupiravir, respectively. However, their effects are limited and some side effects are observed in patients based on the use of these drugs. This makes patient compliance difficult and reduces the effectiveness of the treatment.

From this point of view, in studies carried out to reduce and eliminate the effects of COVID-19 disease, research for single-multitarget molecules continues. Unfortunately, "single-target molecule" approach has not been effective in the treatment of diseases with complex mechanisms.

Although all these drugs, whose research is ongoing, have shown antiviral effects against the coronavirus, there is no drug with a proven effect in the treatment of COVID-19 patients that can eliminate the effects of the ongoing COVID-19 epidemic.

Bozek and Winterstein, (Journal of Asthma, 2020) discloses a retrospective observational study conducted on elderly asthmatic patients, demonstrating that the rate of confirmed COVID-19 infection was significantly lower in the group treated with montelukast compared to the control group.

Abu-Saleh et al., (Physical Chemistry Chemical Physics, 2020) discloses a ligand-based and structure-based virtual screening study targeting the SARS-CoV-2 Main Protease (Mpro).

Maffucci and Contini, (Journal of Proteome Research, 2020) discloses a comprehensive in silico virtual screening study covering more than 3000 approved drugs, identifying montelukast within the top 2% of compounds against both the Mpro and the Spike/ACE-2 proteins of SARS-CoV-2.

Almerie and Kerrigan, (Medical Hypotheses, 2020) discloses a hypothesis proposing montelukast as a potential therapeutic candidate for COVID-19, encompassing both direct antiviral interference with viral replication and suppression of heightened cytokine release, particularly in patients with obesity and metabolic syndrome.

Therefore, there is an urgent need for new treatment approaches for use in the treatment, relieving symptoms, delaying or preventing the progression of COVID-19 disease.

In addition, there is a search for a therapeutically safe treatment method that can inactivate the SARS-CoV-2 virus by preventing the replication and binding of the SARS-CoV-2 virus to the cells, and that does not cause side effects in patients.

### Objects of the Invention

Principal object of the present invention is to provide a montelukast derivative for use in the prevention of COVID-19 disease, relief of its symptoms, delay of its progression, and treatment thereof.

Another object of the present invention is to inactivate the SARS-CoV-2 viruses that cause COVID-19 disease. In order to inactivate the virus, the binding of the SARS-CoV-2 virus to the cell and its replication is to be prevented. In this case, it is intended to provide dual inhibition of Main Protease (Mpro) and Spike/ACE-2 proteins, which are the main targets of SARS-CoV-2 virus.

Another object of the present invention is to provide a montelukast derivative for use in the treatment of COVID-19 disease with minimal side effects. Another object of the present invention is to have a high level of patient compliance throughout the treatment in patients diagnosed with COVID-19 or in persons with symptoms of COVID-19 disease.

Another object of the present invention is to provide a montelukast derivative showing a higher therapeutic effect compared to the drugs used in the prior art. Still another object of the present invention is to provide a montelukast derivative for use in the treatment of COVID-19 disease, as well as for use in the prevention thereof in persons at risk.

### Summary of the Invention

The present invention discloses a montelukast derivative for use in dual inhibition of Main Protease and Spike/ACE-2 proteins in SARS-CoV-2 virus.

According to the present invention, the montelukast derivative may be 4-[(2R)-2-carboxylato-2-[(2S)-4-{[(1R)-1-{3-[(1E)-2-(7-chloroquinoline-2)-yl)ethyl]phenyl}-3-[2-(2-hydroxypropan-2-yl)phenyl]propyl]azaniumyl}-2-hydroxybutanamido]ethyl]-1H-imidazole-3-ium.

According to another embodiment of the invention, the montelukast derivative may be (1R,3R)-3-carbamoyl-1-[(1R)-1-{3-[(1E)-2-(7-chloroquinoline-2-yl)ethenyl]phenyl}-3-[4-(2-hydroxypropan-2-yl)phenyl]propyl]-4-methylpiperazine-1-ium.

According to another embodiment of the invention, the montelukast derivative may be 2-{[(1R)-1-{3-[(1E)-2-(7-chloroquinoline-2-yl)ethenyl]phenyl}-3-[2-(2-hydroxypropan-2-yl)phenyl] propyl]amino}-9-[(2-hydroxyethoxy)methyl]-6,9-dihydro-1H-purine-6-one.

According to another embodiment of the invention, the montelukast derivative may be 4-{[(1R)-1-{3-[(1E)-2-(7-chloroquinoline-2-yl)ethyl]phenyl}-3-[2-(2-hydroxypropan-2-yl)phenyl] propyl]sulfanyl}-N-methyl-3-oxobutanamide.

According to the invention, in the dual inhibition of Main Protease and Spike/ACE-2 proteins in SARS-CoV-2 virus, sodium salt of the montelukast derivative can be used.

According to the present invention, a montelukast derivative for use in inhibiting the replication of SARS-CoV-2 virus.

According to another embodiment, a montelukast derivative for use in inhibiting the entry of SARS-CoV-2 virus into healthy cells. Furthermore, said molecule for use in inhibiting viral or retroviral replication of the SARS-CoV-2 virus.

According to the invention, a montelukast derivative is for use in the prevention , relief of symptoms, delay of progression and/or treatment of COVID-19 disease caused by the SARS-CoV-2 virus.

Also, the use of a montelukast derivative for the prevention, relief of symptoms, delay of progression and/or treatment of pneumonia infection caused by the SARS-CoV-2 virus is also disclosed.

According to the invention, a montelukast derivative for use in the prevention, relief of symptoms, delay of progression, and/or treatment of fever, shortness of breath, distress syndrome, cough, musculoskeletal pain, or a combination thereof, caused by the SARS-CoV-2 virus.

In studies conducted to prove the effect against the SARS-CoV-2 virus, montelukast at a concentration of 100 micromolar inhibits Mpro protein by 74%. Also, according to the invention, the inhibition of said molecule of the Spike/ACE-2 interface interaction is 54.04 micromolar.

According to the invention, the daily dose of a montelukast derivative is in the range of 5-120 mg/day, preferably in the range of 10-60 mg/day and more preferably in the range of 10-30 mg/day.

According to the invention, a montelukast derivative is administered at least once, at most three times a day.

The present invention also relates to a pharmaceutical formulation containing a montelukast derivative as an active ingredient for dual inhibition of Main Protease and Spike/ACE-2 proteins in SARS-CoV-2 virus, and at least one pharmaceutically acceptable excipient.

According to the invention, said pharmaceutical formulation comprises a montelukast derivative as defined in claim 1, and at least one pharmaceutically acceptable excipient.

According to another embodiment of the invention, the montelukast derivative in said pharmaceutical formulation may be 4-[(2R)-2-carboxylato-2-[(2S)-4-{[(1R)-1-{3-[(1E)-2-(7-chloroquinoline-2)-yl)ethyl]phenyl}-3-[2-(2-hydroxypropan-2-yl)phenyl]propyl]azaniumyl}-2-hydroxybutanamido]ethyl]-1H-imidazole-3-ium.

In another embodiment of the invention, the montelukast derivative in said pharmaceutical formulation may be (1R,3R)-3-carbamoyl-1-[(1R)-1-{3-[(1E)-2-(7-chloroquinoline-2-yl)ethenyl]phenyl}-3-[4-(2-hydroxypropan-2-yl)phenyl]propyl]-4-methylpiperazine-1-ium.

According to another embodiment of the invention, the montelukast derivative in said pharmaceutical formulation may be 2-{[(1R)-1-{3-[(1E)-2-(7-chloroquinoline-2-yl)ethenyl]phenyl}-3-[2-(2-hydroxypropan-2-yl)phenyl]propyl]amino}-9-[(2-hydroxyethoxy)methyl]-6,9-dihydro-1H-purine-6-one.

In another embodiment of the invention, the montelukast derivative in said pharmaceutical formulation may be 4-{[(1R)-1-{3-[(1E)-2-(7-chloroquinoline-2-yl)ethyl]phenyl}-3-[2-(2-hydroxypropan-2-yl)phenyl]propyl]sulfanyl}-N-methyl-3-oxobutanamide.

In another embodiment of the invention, sodium salt of the montelukast derivative is used in said pharmaceutical formulation.

According to the present invention, said pharmaceutical formulation may be administered orally, nasally, topically, parenterally or by inhalation.

According to another embodiment of the present invention, said pharmaceutical formulation may be in the form of tablets, capsules, pellets, granules, solutions, parenteral solutions, syrups, dry powders, creams, gels, sprays or emulsions.

According to the invention, said pharmaceutical formulation is administered to the patient for 5-20 days, preferably 8-15 days.

According to another embodiment of the invention, said pharmaceutical formulation can be used in individuals previously diagnosed with a chronic respiratory disease such as asthma, COPD; diabetes; and/or heart disease.

According to the invention, said pharmaceutical formulation can be used immediately after the onset of symptoms of COVID-19 disease caused by the SARS-CoV-2 virus.

In another embodiment of the invention, said pharmaceutical formulation can be used in patients who have previously received a treatment for COVID-19 disease but have failed to respond.

According to another embodiment of the invention, said pharmaceutical formulation can be used in patients for whom no medication has been previously administered for the treatment of COVID-19 disease. In another embodiment of the invention, said pharmaceutical formulation can be used for protection from COVID19 disease in persons at risk who do not have any symptoms related to COVID-19 disease.

In another embodiment of the invention, the pharmaceutical formulation may contain at least one additional active ingredient.

According to the invention, said additional active ingredient may be favipiravir, arbidol, camostat mesylate, ritonavir, lopinavir, GC-376, or a combination thereof.

In an embodiment of the present invention, said pharmaceutical formulation also contains the active ingredient favipiravir.

In another embodiment of the invention, the active ingredients in the formulation can be administered simultaneously, sequentially or separately.

In another embodiment of the invention, the active ingredients in the formulation can be formulated in a single dosage form.

In another embodiment of the present invention, said pharmaceutical formulation may be formulated as a bilayer tablet.

In another embodiment of the present invention, the active ingredients in said formulation can be present in different layers.

In another embodiment of the invention, an inert layer may be present between the layer containing a montelukast derivative and the layer containing the additional active ingredient.

In another embodiment of the invention, the active ingredients in the formulation can be formulated to be in different formulations.

According to another embodiment of the invention, said different pharmaceutical formulations may be in the same dosage form or in different dosage form.

According to the invention, said different pharmaceutical formulations may be in the same kit package.

### Brief Description of the Drawings

The drawings, whose brief description is provided below are just given for better understanding of the present invention and as such, are not intended to determine the scope of the claimed subject matter, in the absence of the description.
Figure 1 shows a representative complex structure of Montelukast in the SARS-CoV-2 Mpro binding pocket.
Figure 2 shows significant interactions of Montelukast during molecular dynamics (MD) simulations initiated by molecular docking (with non-covalent docking pose) in SARS-CoV-2 Mpro.
Figure 3 shows a representative complex structure of Montelukast in the SARS-CoV-2 Mpro binding pocket obtained from the trajectory frames recorded in the MD simulations.
Figure 4 shows significant interactions of Montelukast during molecular dynamics (MD) simulations initiated by molecular docking (with covalent docking pose) in SARS-CoV-2 Mpro.
Figure 5 shows a representative structure of Montelukast at the SARS-CoV-2 Spike/ACE-2 interface.
Figure 6 shows significant interactions of Montelukast during MD simulations initiated by molecular docking (non-covalent docking) pose in SARS-CoV-2 Spike / ACE-2.
Figure 7 shows analysis results of the mean MM / GBSA binding free energy of Montelukast in SARS-CoV-2 Mpro (left) and Spike / ACE-2 (right) targets during the MD simulation.
Figure 8 shows 3CL Protease activity (left) at varying concentrations in the presence of Montelukast; and a dose-response curve (right) of Montelukast versus 3CL protease.
Figure 9 shows solvent correction (left) in DMSO from 10% to 8.6%; 1: 1 steady-state binding pattern of Montelukast in 3CL protease (right, top); subtracted and correction sensograms (right, bottom) of Montelukast binding curves for 3CL protease.
Figure 10 shows cell viability upon exposure of HEK293T, Vero E6, Calu-3 and A549 to Montelukast in the concentration range of 1 to 200 µM.
Figure 11-A shows inhibition of entry of HEK293T/hACE2 cells.
Figure 11-B is representative images of Cell viability and neutralization.
Figure 12 shows a study of a real-time cell analyzer for the effect of the Montelukast molecule on VERO E6 cells for 150 hours without the addition of virus.
Figure 13 shows a study of a real-time cell analyzer for the neutralizing activity of the Montelukast molecule against the SARS-CoV-2 virus for 150 hours.
Figure 14 shows a comparison of CIT50 values for different concentrations of Montelukast.
Figure 15 shows a comparison of the CIT50 values of a study on the SARS-CoV-2 virus neutralization efficiency obtained with different concentrations of RTCA.

### Detailed Description of the Invention

The present invention defines a montelukast derivative for use in the dual inhibition of Main Protease and Spike/ACE-2 proteins in the SARS-CoV-2 virus. According to the invention, the montelukast derivative inactivates the SARS-CoV-2 virus and prevents its replication. In addition, it is observed that the binding of SARS-CoV-2 virus to healthy cells is inhibited by the use of a montelukast derivative.

There is a large body of research aimed at treating COVID-19 disease. While some of them cover vaccine studies, some includes approved molecules used for different indications. In *in silico* drug screening studies, the behavior of approximately 7000 active ingredients, which are FDA-approved or in clinical phase, against SARS-CoV-2 target proteins are investigated.

The most important target proteins in the entry into cell and replication of SARS-CoV-2 virus are known as main protease (Mpro), TMPRSS2, Spike/ACE2 and RNA-dependent-RNA polymerase (RDRP). With virtual screening results for Main protease and Spike/ACE-2 proteins in SARS-CoV-2 virus, it was determined that the active ingredient Montelukast strongly binds to MPro and Spike/ACE-2 targets. After the strong binding results at the Mpro and Spike/ACE-2 interface, the effect of montelukast against SARS-CoV-2 virus was investigated with both multi-scale molecular modeling approaches and 4 different *in vitro* experiments. Results of FRET-based biochemical tests, surface plasmon resonance (SPR), pseudovirus neutralization and virus neutralization experiments demonstrated the effect of Montelukast on SARS-CoV-2. FRET-based biochemical tests were performed against the main protease, and pseudovirus neutralization experiments were performed against HEK293T/hACE2.

Montelukast is a selective leukotriene (LTD₄) receptor antagonist which is a member of the quinolines, and it was first approved by the FDA as an oral tablet in 1998. It is an active ingredient used in the treatment of diseases such as perennial allergic rhinitis, seasonal allergic rhinitis and asthma. The Singulair^{®} containing montelukast sodium is prescribed for the treatment of allergic asthma and allergic rhinitis in children aged 15 years and older and adults.

A representation of the chemical structure of montelukast, IUPAC nomenclature "(R,E)-2-(1-((1-(3-(2-(7-Chloroquinoline-2-yl)vinyl)phenyl)-3-(2-(2-hydroxypropan-2-)yl)phenyl)propylthio)methyl)cyclopropyl)acetic acid" is depicted in Formula I.

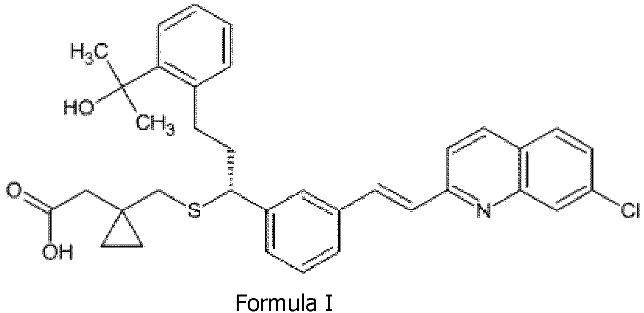

Montelukast, which acts as an anti-inflammatory agent, is known to have an effect on reducing the frequency and severity of wheezing that occurs after upper respiratory tract infection caused by adenovirus, influenza, metapneumovirus, and coronavirus. In other words, montelukast is considered to relieve upper respiratory tract symptoms in these patients.

Montelukast exhibits anti-inflammatory effect thanks to bradykinin and leukotriene antagonism; it has also been found to be effective in mechanisms involving the activity of ACE receptors. Since the SARS-CoV-2 virus enters the cell via ACE receptors and some of the cases result in death due to excessive inflammation, montelukast is predicted to contribute to the treatment of COVID-19.

In studies to prove the effect of montelukast against the SARS-CoV-2 virus, it has been shown that montelukast binds to Main Protease and inhibits SARS-CoV-2 activity by 74% at a concentration of 100 micromolar. Accordingly, in Main Protease, the IC₅₀ value of montelukast was determined to be 28.36 micromolar.

In this context, the dual inhibition profile of Montelukast was investigated through multi-scale molecular modeling studies. Subsequently, the effects of montelukast on both targets were characterized by different *in vitro* experiments, including Fluorescent Resonance Energy Transfer (FRET)-based Main protease enzyme inhibition assay, surface plasmon resonance (SPR) spectroscopy, pseudovirus neutralization and virus neutralization on HEK293T / hACE2. *In silico* and *in vitro* results confirmed the dual effect of montelukast on both host cell virus entry (Spike/ACE-2) and Main protease enzyme inhibition.

In this context, the present invention relates to a montelukast derivative for use in dual inhibition of Main Protease and Spike/ACE-2 proteins in SARS-CoV-2 virus.

Montelukast derivatives according to the invention are montelukast analogs obtained by addition of small fragments, as listed in Table 1.

**Table 1. Montelukast Analogs.**

| | |
|---|---|
| 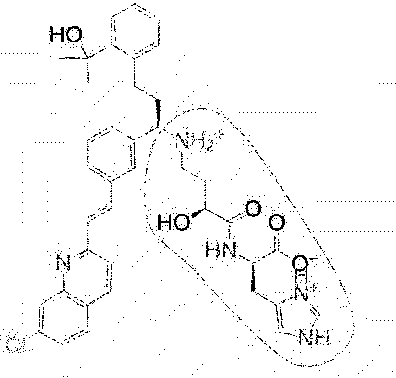 | 4-[(2R)-2-carboxylato-2-[(2S)-4-{[(1R)-1-{3-[(1E)-2-(7-chloroquinoline-2)-yl)ethyl]phenyl}-3-[2-(2-hydroxypropan-2-yl)phenyl]propyl]azaniumyl}-2-hydroxybutanamido]ethyl]-1H-imidazole-3-ium |
| 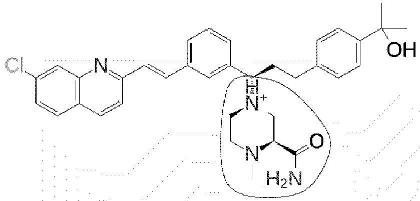 | (1R,3R)-3-carbamoyl-1-[(1R)-1-{3-[(1E)-2-(7-chloroquinoline-2-yl)ethenyl]phenyl}-3-[4-(2-hydroxypropan-2-yl)phenyl]propyl]-4-methylpiperazine-1-ium |
| 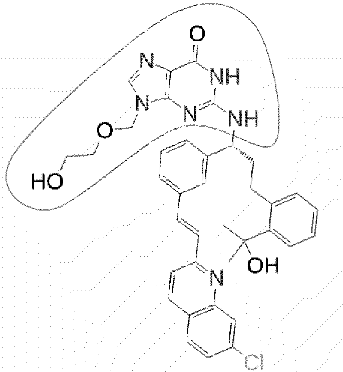 | 2-{[(1R)-1-{3-[(1E)-2-(7-chloroquinoline-2-yl)ethenyl]phenyl}-3-[2-(2-hydroxypropan-2-yl)phenyl] propyl]amino}-9-[(2-hydroxyethoxy)methyl]-6,9-dihydro-1H-purine-6-one |
| 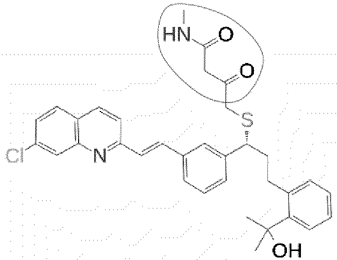 | 4-{[(1R)-1-{3-[(1E)-2-(7-chloroquinoline-2-yl)ethyl]phenyl}-3-[2-(2-hydroxypropan-2-yl)phenyl]propyl]sulfanyl}-N-methyl-3-oxobutanamide |

In Table 1, the small fragments added for the montelukast analogues are shown in the molecular form.

According to another embodiment of the invention, the montelukast derivative inhibits the replication of SARS-CoV-2 virus.

As used here, the terms "inhibit", "inhibition" refer to a significant reduction of the basic activity of the biological activity or mechanism causing a particular disease, and accordingly, prevention of the survival and/or growth of the virus population or reducing the growth rate.

Furthermore, a montelukast derivative is for use in inhibiting the entry of SARS-CoV-2 virus into healthy cells. Besides, a montelukast derivative is for use in inhibiting viral or retroviral replication of the SARS-CoV-2 virus.

The active ingredient of the invention can bind to the important targets of the SARS-CoV-2 virus responsible for the COVID-19 disease. With the binding of the virus to the Spike/ACE-2 interface, the virus is prevented from entering the cell, and by inactivating the Main Protease, the maturation and replication of the virus in the cell is prevented. In this case, an effective therapeutic approach for COVID-19 disease is obtained. Said two target proteins are among the proteins with the most critical role in the spread of SARS-CoV-2.

As described herein, the term 'montelukast derivative' refers to analogs of montelukast obtained by the addition of small fragments, as listed in Table 1.

According to the present invention, the montelukast derivative is for use in the prevention , relief of symptoms, delay of progression and/or treatment of COVID-19 disease caused by the SARS-CoV-2 virus.

According to the invention, symptoms in COVID-19 disease are sore throat, fever, cough, shortness of breath, respiratory failure, heart failure, pneumonia, muscle-joint pains, multi-organ failure and/or pneumonia.

As described herein, "treat" refers to the elimination of the COVID-19 disease or the mitigation of the effect of at least one of the symptoms associated with it. The expected physical recovery here is physiological recovery or both physical and physiological recovery.

As used herein, "prevention of disease" refers to the use of said composition to ensure that the disease does not appear at all in persons who have not been diagnosed with COVID-19 positive or who have not yet exhibited any symptoms. As described herein, the montelukast derivative for the prevention of the disease, i.e., in order to ensure that the SARS-CoV-2 virus has no effect on healthy cells, for persons who do not exhibit symptoms of COVID-19 but are in the risk group.

The "risk group" mentioned herein specifically includes adults over the age of 65.

According to the present invention, the symptoms, at least one of which is desired to be eliminated by inhibiting Mpro and Spike/ACE-2 proteins in the SARS-CoV-2 virus, are sore throat, fever, cough, shortness of breath, respiratory failure, heart failure, pneumonia, muscle-joint pains or multi-organ failure.

According to a preferred embodiment of the invention, the montelukast derivative is for use in the prevention, relief of symptoms, delay of progression and/or treatment of pneumonia infection caused by the SARS-CoV-2 virus.

According to another preferred embodiment of the invention, the montelukast derivative is for use in the prevention, relief of symptoms, delay of progression, and/or treatment of fever, shortness of breath, distress syndrome, cough, muscle-joint pain, or a combination thereof, caused by the SARS-CoV-2 virus.

According to the invention, the montelukast derivative inhibits the Mpro protein in the SARS-CoV-2 virus by 74% at a concentration of 100 micromolar. The term "inhibit" as used herein refers to the rate at which Mpro enzyme activity decreases. According to the invention, inhibition of the montelukast derivative of the Spike/ACE-2 interface interaction in the SARS-CoV-2 virus was measured to be 54.04 micromolar. Here, "interface interaction inhibition" means "50% effective neutralization concentration". In the main protease target structure, montelukast has an IC₅₀ value of 28.36 micromolar.

According to a preferred embodiment of the invention, the daily dose is in the range of 5-120 mg/day, preferably 10-60 mg/day and more preferably in the range of 10-30 mg/day, for dual inhibition of Mpro and Spike/ACE-2 proteins in the SARS-CoV-2 virus. The optimum daily dose required to achieve maximum dual inhibition of Mpro and Spike/ACE-2 proteins has been calculated to be 55 mg (bioavailability of montelukast being 66%). The daily dose of the active ingredient of the invention is an amount necessary or sufficient to treat, prevent, relieve symptoms or delay the progression of the viral infection as described herein, and the symptoms caused by SARS-CoV-2 virus. The amount of daily dose may also be defined as an amount of active substance, which is the lowest dose required to achieve the desired therapeutic effect.

According to another embodiment of the invention, the montelukast derivative is administered at least once, at most three times a day, in order to dual inhibit Mpro and Spike/ACE-2 proteins in the SARS-CoV-2 virus.

The present invention also relates to a pharmaceutical formulation containing the montelukast derivative as an active ingredient for dual inhibition of Main Protease and Spike/ACE-2 proteins in SARS-CoV2 virus, and at least one pharmaceutically acceptable excipient.

Pharmaceutical formulations according to the invention comprise a montelukast derivative as defined in claim 1, and at least one pharmaceutically acceptable excipient.

In another preferred embodiment of the invention, said pharmaceutical formulation may contain montelukast analogs obtained by the addition of small fragments. Table 1 lists the montelukast derivatives according to the invention. The "montelukast analogue" as referred to herein includes molecules obtained by adding small fragments to montelukast. The term "montelukast analogue" may be replaced by the term "montelukast derivative".

According to the invention, the montelukast derivative may be 4-[(2R)-2-carboxylato-2-[(2S)-4-{[(1R)-1-{3-[(1E)-2-(7-chloroquinoline-2)-yl)ethyl]phenyl}-3-[2-(2-hydroxypropan-2-yl)phenyl]propyl]azaniumyl}-2-hydroxy butanamido]ethyl]-1H-imidazole-3-ium.

According to another embodiment of the invention, the montelukast derivative may be (1R,3R)-3-carbamoyl-1-[(1R)-1-{3-[(1E)-2-(7-chloroquinoline-2-yl)ethenyl]phenyl}-3-[4-(2-hydroxypropan-2-yl)phenyl]propyl]-4-methylpiperazine-1-ium.

According to another embodiment of the invention, the montelukast derivative may be 2-{[(1R)-1-{3-[(1E)-2-(7-chloroquinoline-2-yl)ethenyl]phenyl}-3-[2-(2-hydroxypropan-2-yl)phenyl] propyl]amino}-9-[(2-hydroxyethoxy)methyl]-6,9-dihydro-1H-purine-6-one.

According to another embodiment of the invention, the montelukast derivative may be 4-{[(1R)-1-{3-[(1E)-2-(7-chloroquinoline-2-yl)ethyl]phenyl}-3-[2-(2-hydroxypropan-2-yl)phenyl] propyl]sulfanyl}-N-methyl-3-oxobutanamide.

In another preferred embodiment, in the dual inhibition of Main Protease and Spike/ACE-2 proteins in SARS-CoV2 virus, sodium salt of the montelukast derivative can be used.

According to the invention, the pharmaceutical formulation used to inhibit Mpro and Spike/ACE-2 proteins in the SARS-CoV-2 virus that causes COVID-19 disease can be administered orally, nasally, topically, parenterally or by inhalation.

In another embodiment of the present invention, said pharmaceutical formulation may be in the form of tablets, capsules, pellets, granules, solutions, parenteral solutions, syrups, dry powders, creams, gels, sprays or emulsions.

In order to treat, relieve symptoms, delay or prevent the progression of COVID-19 disease, said pharmaceutical compositions can be administered to the patient for 5-20 days, preferably 8-15 days.

According to the invention, said pharmaceutical formulation is used in individuals previously diagnosed with a chronic respiratory disease such as asthma, COPD; diabetes; and/or heart disease.

According to the invention, said pharmaceutical formulation can be used immediately after the onset of symptoms of COVID-19 disease caused by the SARS-CoV-2 virus.

In another embodiment of the invention, said pharmaceutical formulation is used in patients who have previously received a treatment for COVID-19 disease but have failed to respond.

In another embodiment of the invention, said pharmaceutical formulation is used in patients for whom no medication has been previously administered for the treatment of COVID-19 disease.

In another embodiment of the invention, said pharmaceutical formulation is used for protection from COVID-19 disease in persons at risk who do not have any symptoms related to COVID-19 disease.

According to the present invention, the montelukast derivative used to dual inhibit Mpro and Spike/ACE2 proteins in SARS-CoV-2 virus can be used together with at least one additional active ingredient. According to the invention, said active ingredient may be arbidol, camostat mesylate, ritonavir, lopinavir, GC-376, favipiravir, or a combination thereof. In the preferred embodiment, the additional active ingredient is favipiravir.

The chemical name of the molecule "GC-376" defined herein is (25)-2-[(25)-2-{[(benzyloxy)carbonyl]amino}-4-methylpentanamido]-1-hydroxy-3-(2-oxopyrrolidine-3-yl)propane-1-sulfonic acid.

In the present invention, by combining the active ingredients a montelukast derivative and favipiravir, a higher therapeutic benefit resulting from a synergistic effect can be achieved compared to using said active ingredients separately.

According to the invention, the montelukast derivative and at least one additional active ingredient can be administered simultaneously, sequentially or separately. The active ingredients in the pharmaceutical formulation can be in a single dosage form or in different formulations of the active ingredients.

In case the active ingredients are formulated in the same formulation, drug-drug interactions may occur because of the combination of the active ingredients. During the shelf life, the montelukast derivative may interact with the additional active ingredient, resulting in formation of impurities. In this case, the desired benefit from the drug is not obtained, since the desired dose cannot be administered to the patient. In order to prevent drug interactions, the montelukast derivative and additional active ingredient are formulated in separate layers.

According to the invention, the bilayer tablet formulation contains a montelukast derivative and favipiravir.

According to another embodiment of the invention, in pharmaceutical formulation where the montelukast derivative and additional active ingredient are in separate layers, an inert layer is present between said layers. In this way, the desired stability can be maintained throughout the storage life of the final product.

According to another embodiment of the invention, between the montelukast derivative-containing and favipiravir-containing layers, there is an inert layer where there is no active ingredient.

In another embodiment of the invention, where a montelukast derivative and additional active ingredient are formulated in different formulations, the different formulations may be in the same dosage form or in different dosage forms.

In addition, the montelukast derivative formulation and additional active ingredient formulation in said pharmaceutical formulation can be used in accordance with the use of the kit.

### EXAMPLES:

In the following examples, montelukast is used as a reference compound. The tests described below is for multiscale molecular modeling of the dual inhibition profile of montelukast. The effect of montelukast on both targets was characterized by different *in vitro* experiments, including Fluorescent Resonance Energy Transfer (FRET)-based Main protease enzyme inhibition assay, surface plasmon resonance (SPR) spectroscopy, pseudovirus neutralization and virus neutralization on HEK293T / hACE2, using the xCELLigence MP real-time cell analyzer.

### - In Silico Drug Repurposing Studies:

Both non-covalent and covalent docking approaches were performed in the SARS-CoV-2 Mpro target structure.

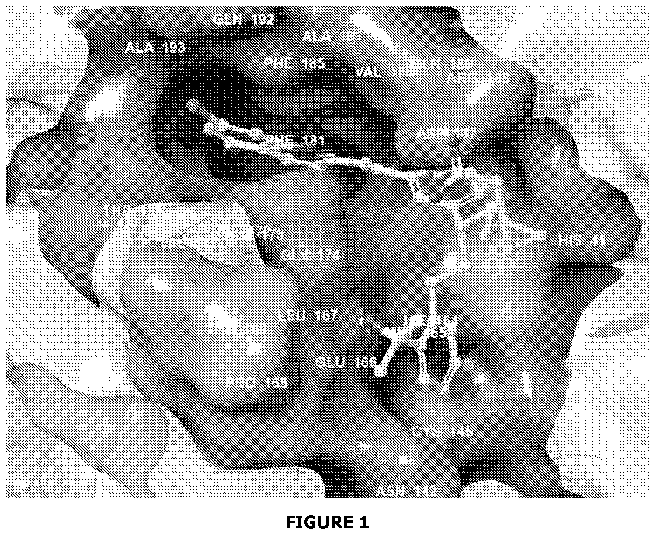

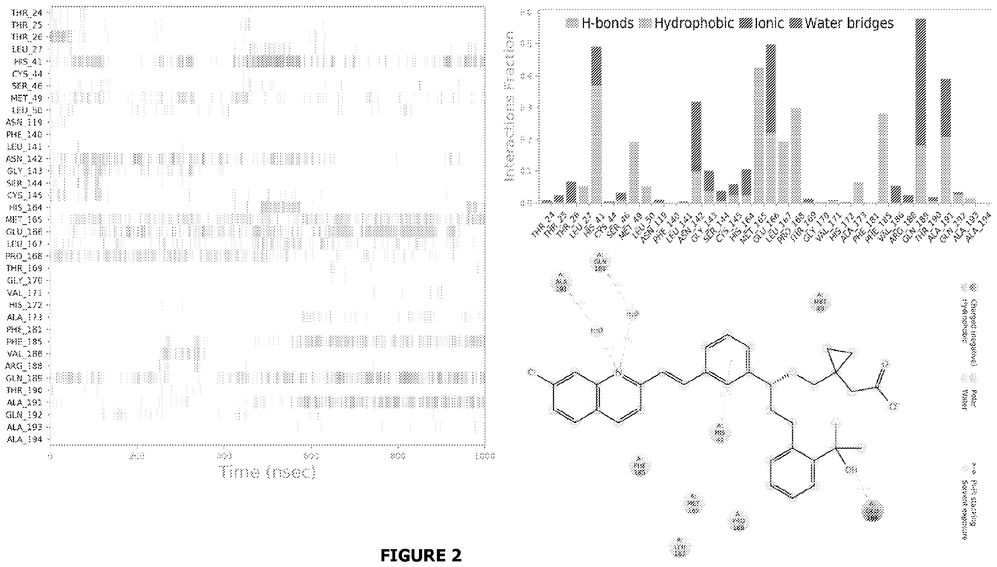

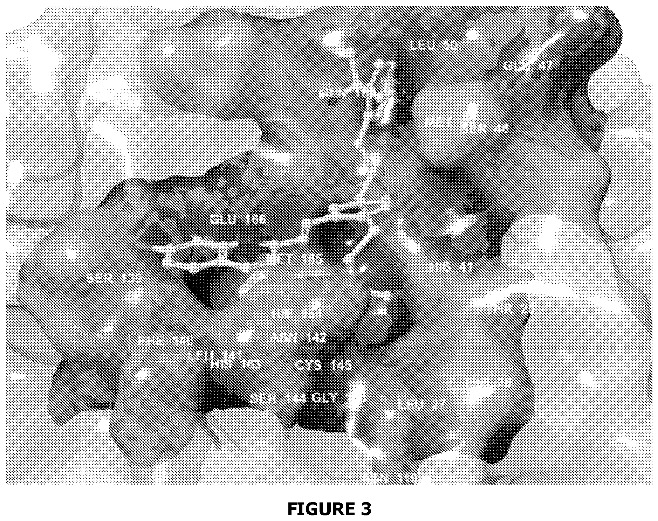

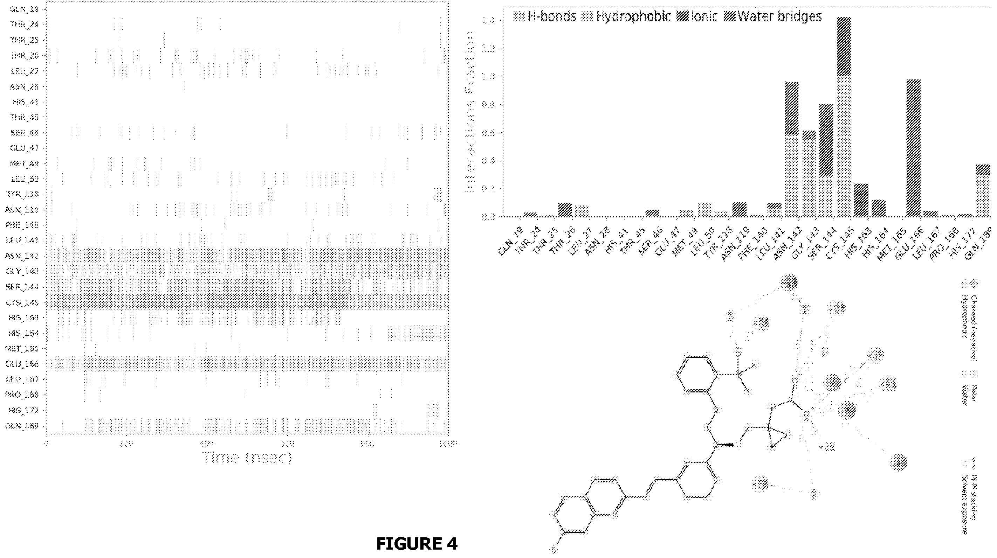

Figure 1 shows a representative structure of Montelukast on the SARS-CoV-2 Mpro target obtained from trajectory frames recorded during molecular dynamics (MD) simulations initiated from non-covalent docking. Significant residues in ligand interaction were detected to be His41, Met49, Asn142, Met165, Glu166, Leu167, Pro168, Phe185, Gln189 and Ala191. (Figure 2). Here, protein interactions with montelukast are monitored throughout the simulation. Stacked bar charts are normalized along the trajectory frame. For example, a value of 0.5 indicates that specific interaction for 50% of the simulation time is preserved. In Figure 3, a surface view of the representative complex of Montelukast combined with Mpro shows important interactions therein. The amino acid residues important in the corresponding interactions from simulations initiated by covalent docking are Asn142, Gly143, Ser144, Cys145, His164, Glu166, and Gln189 (Figure 4).

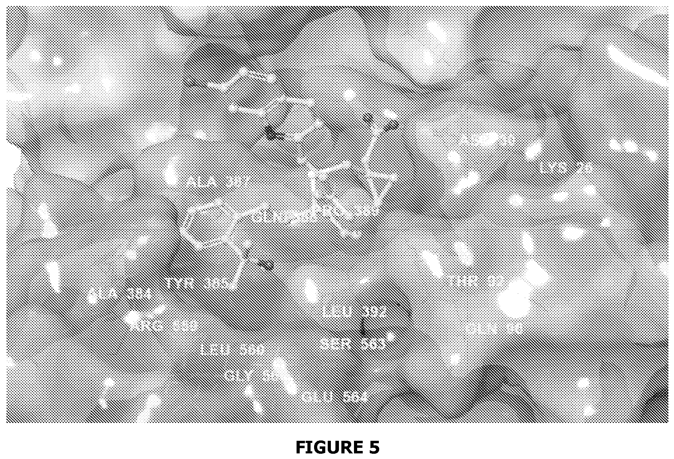

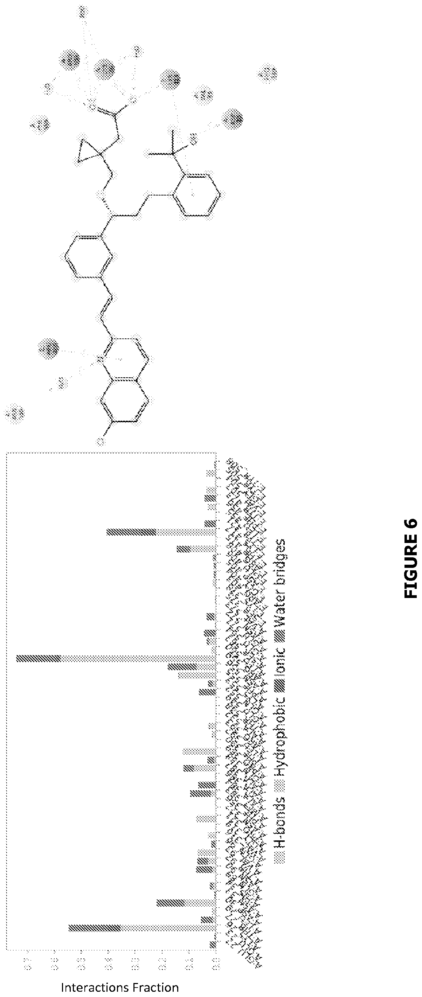

The effect of montelukast in the SARS-CoV-2 Spike/ACE-2 region was also investigated. A docking pose of montelukast at the Spike/ACE-2 interface was used in MD simulations. Figures 5 and 6 represent 3D and 2D ligand interaction diagrams. In Figure 6, stacked bar graphs are normalized along the trajectory frame. For example, a value of 0.5 indicates that specific interaction for 50% of the simulation time is preserved.

Significant residues in ligand interactions were detected to be Lys26, Asp30, Val93, Pro389, Arg408, Lys417, Phe555, Asn556 and Arg559. The binding energies of montelukast on Mpro and Spike/ACE-2 targets were also investigated.

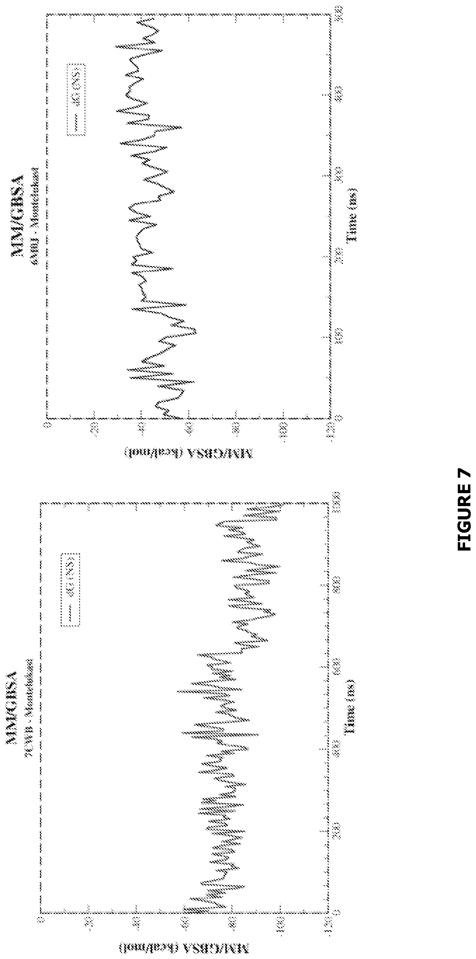

The mean Molecular Mechanics/Generalized Born Surface Area (MM / GBSA) binding energy at the Mpro ligand binding site of montelukast was measured as -79.60 ± 8.66 kcal/mol in 1 microsecond length simulations. This value was maintained when the simulation was extended 3 times (3 microseconds) (-84.86± 9.22 kcal/mol). In Spike/ACE-2, this value was calculated as -43.93 ± 7.66 kcal/mol. (Figure 7)

### - Mpro enzyme inhibition test based on Fluorescence Resonance Energy Transfer (FRET)

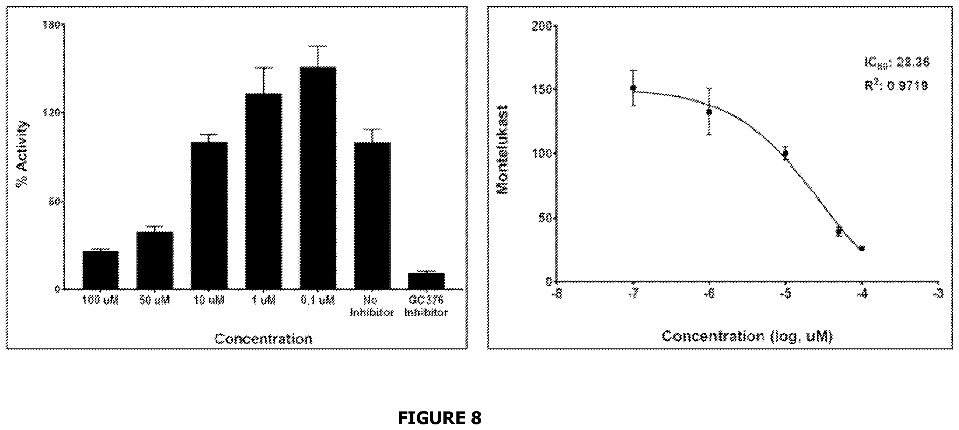

The Mpro experiment revealed that the Montelukast molecule inhibited the activity of main protease enzyme in a concentration-dependent manner. The maximum inhibitory concentration was set to 100 µM and inhibited 74.04% of the enzyme activity. At 50 µM concentration, inhibition was calculated as 60.59% (Figure 8). GC376 was used as a positive control molecule in this assay and showed 88.59% inhibition at 100 µM concentration. In Figure 8 (left), "Inhibitory Activity" indicates the percentage of inhibited 3CL enzyme activity. "No inhibitor" represents 3CL protease activity without any inhibitor, whereas GC376 inhibitor is a broad spectrum antiviral used for comparison.

With the nonlinear regression analysis of the five different concentrations prepared, it was determined that the concentration at which the Montelukast molecule resulted 50% inhibition was 28.36 µM (Figure 8).

### - Surface plasmon resonance (SPR) spectroscopy:

In this study, surface plasmon resonance (SPR) spectroscopy was also used to evaluate the binding kinetics and affinity of this interaction, along with FRET-based analyzes of Montelukast in the SARS-CoV-2 Main protease. SPR devices can be used for drug screening for many diseases, including COVID-19.

Here, after analysis of multi-scale molecular modeling studies and FRET-based Mpro enzyme inhibition experiments, SPR was used to determine the potential role of Montelukast in SARS-CoV-2 infection and its binding kinetics on the Main protease of montelukast.

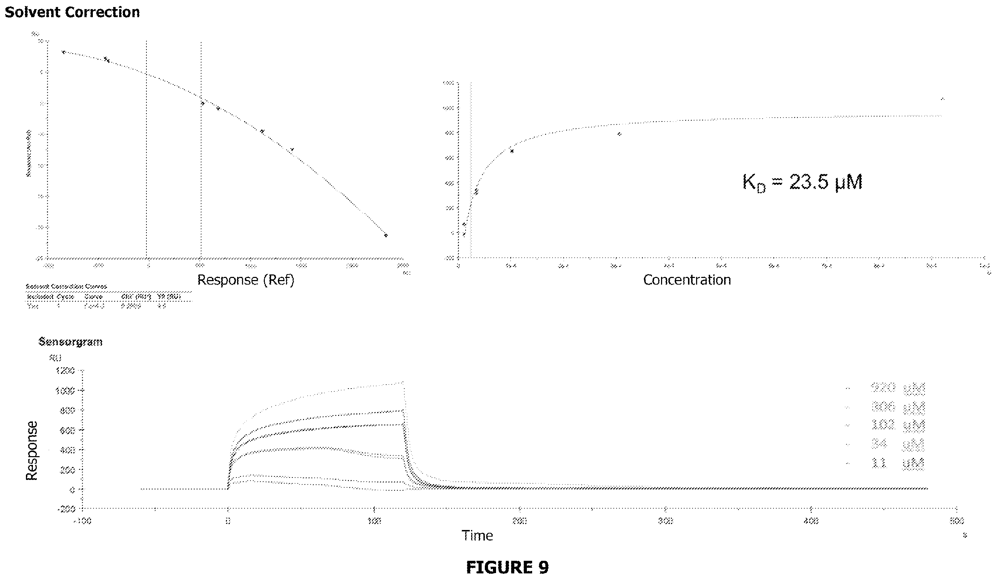

The affinity of montelukast was determined using a 1: 1 steady-state binding affinity to the immobilized 3CL protease. A concentration series ranging from 900 µM to 11 µM at 3-fold dilution was injected onto the immobilized Mpro for a contact time of 60 seconds followed by a dissociation phase of 120 seconds. Concentration values were determined according to preliminary results from binding experiments, in which Montelukast compounds reached the level of binding on the sensor surface. Responses from each Montelukast concentration were plotted against concentration using Biacore T200 evaluation software and evaluated using a 1:1 steady-state binding model. Montelukast was identified as a specific binder for Mpro (Fig. 9). The K_{D} value was measured to be 23.5 µM, which corresponds to the IC₅₀ value determined based on FRET. Considering the binding behavior, Montelukast was found to be fast in both binding and dissociation processes.

### - Neutralization potential of Montelukast against SARS-CoV-2

### Cell Viability:

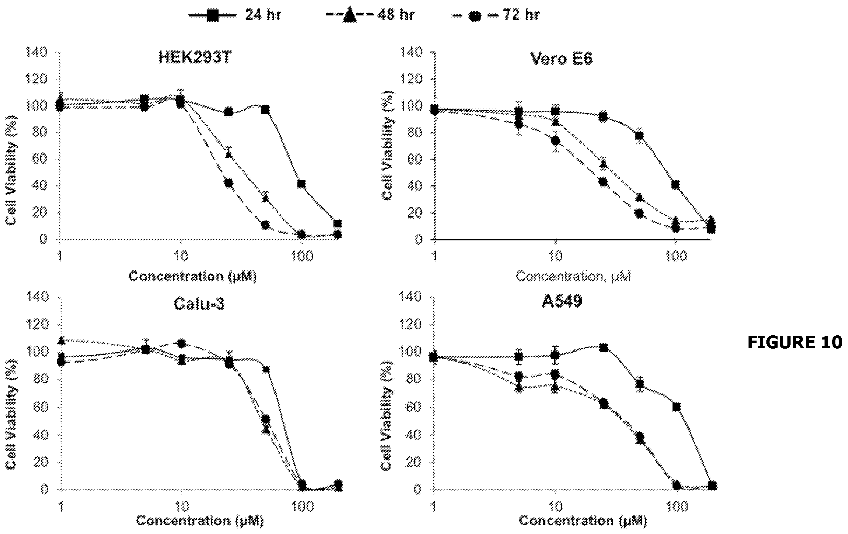

To investigate the dose-response relationship of Montelukast using the HEK293T, Vero E6, Calu-3 and A549 cell lines, cell viability was measured with the MTT calorimetric assay at exposure times of 24, 48 and 72 hours. As shown in Figure 10, HEK293T cell monolayer appeared to be the most sensitive cell line during Montelukast exposure, which is followed by Vero E6, A549 and Calu-3. After 24, 48 and 72 hours of exposure, the IC₅₀ value of Montelukast for the cell lines used is illustrated below Table 2.

**Table 2**

| **IC₅₀ values of Montelukast (µM)** | | | | |
|---|---|---|---|---|
| **Exposure, hr** | **HEK293T** | **Vero E6** | **Calu-3** | **A549** |
| **24** | 92.2 ± 0.8 | 92.39 ± 3.8 | 72.7 ± 0.3 | 117.6 ± 1.4 |
| **48** | 35.3 ± 4.0 | 38.80 ± 2.5 | 47.1 ± 1.3 | 38.2 ± 1.9 |
| **72** | 23.0 ± 0.8 | 28.11 ± 3.1 | 50.7 ± 1.9 | 38.5 ± 0.8 |

Table 2 shows IC50 values of Montelukast relative to the cell lines used, after exposure to Montelukast followed by exposure times of 24, 48 and 72 hours in Vero E6, A549 and Calu-3 cell lines.

The result of the cell viability test allows the determination of concentration ranges for the pseudovirus neutralization test.

### Pseudovirus neutralization

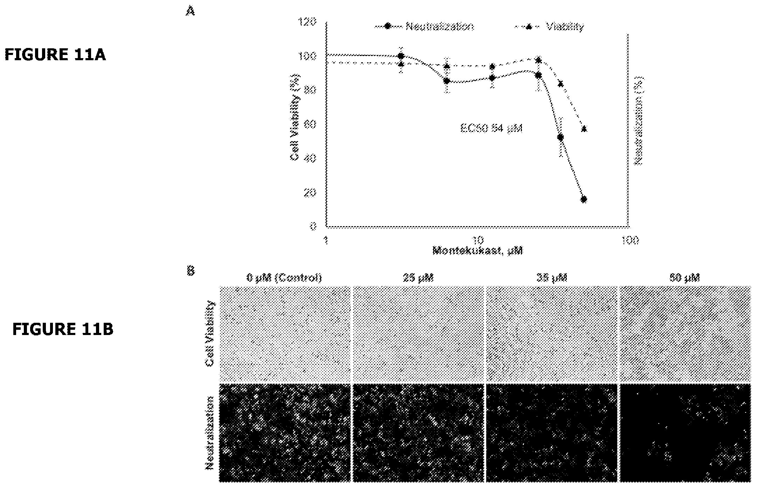

Pseudovirus neutralization on HEK293T / hACE2 was performed using Montelukast at a concentration in the range of 1 to 50 µM (Figure 11).

To evaluate the cell viability at the same concentrations which were used for neutralization, the luciferase activity was measured 72 h post transduction by the CellTiter-Glo Luminescent Cell Viability Assay Kit (Figure 11A; pretreated with different concentrations of Montelukast and transformed with SARS-CoV-2 S pseudovirion) to assess cell viability. The entry efficiency of SARS-CoV-2 pseudoviruses without any treatment was taken as 100%. The representative plug formation at different concentrations was shown in the images taken with 10X magnification during neutralization period (Figure 11-B). The 50% effective neutralization concentration of montelukast was found to be 54.04 µM. Thus, the neutralization potential of montelukast against SARS-CoV-2 was confirmed.

### - Virus Neutralization Assay Using XCELLigence MP Real-Time Cell Analyzer

Neutralization assay was performed based on impedance using xCELLigence MP real time cell analyzer equipment (RTCA), using VERO E6 cells. Said neutralization test was performed based on impedance using XCELLigence MP RTCA. The impedance is expressed as arbitrary units called cell index (CI). According to the results of real-time cell analysis of montelukast without virus incubation for 150 hours, the montelukast molecule is cytotoxic in the absence of virus at a concentration range of 47-375 µM. However, in chronic asthma studies, montelukast was administered to adult patients at a dose up to 200 mg daily for 22 weeks, and in short-term studies at a dose up to 900 mg daily for approximately 1 week, with no clinically significant adverse events. Acute overdose was reported in post-marketing experience and clinical trials with montelukast. These include reports of doses as high as 1000 mg in adults and children (approximately 61 mg/kg in a 42-month-old child). The observed clinical and laboratory findings are consistent with the safety profile in adults and pediatric patients. The majority of the overdose reports have no adverse events. The most common adverse events are consistent with the safety profile of montelukast and include abdominal pain, somnolence, thirst, headache, vomiting, and psychomotor hyperactivity.

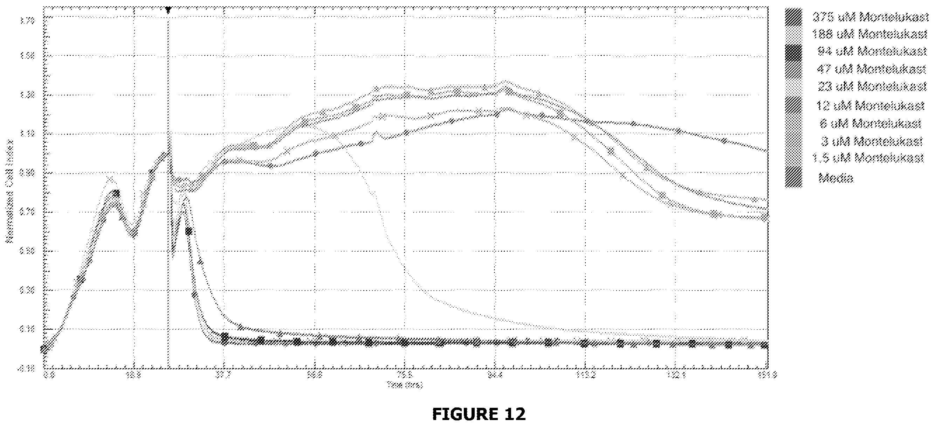

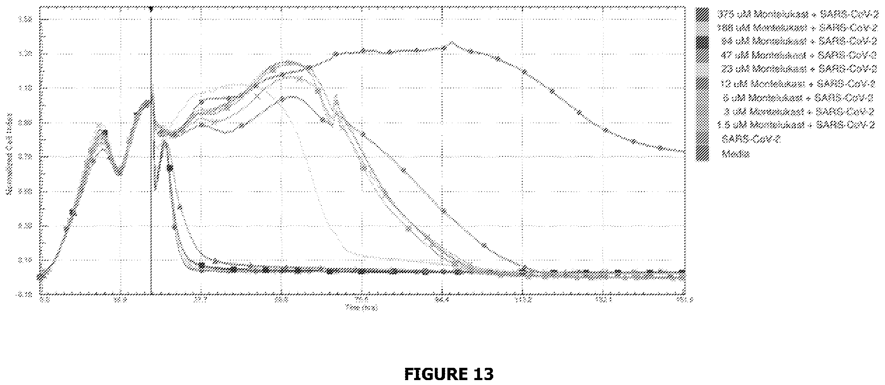

Figure 12 shows that Montelukast can be used up to 23 µM without cytotoxic effects, Figure 13 shows the real-time cell analysis result of Montelukast with SARS-CoV-2. Data were collected for 150 hours at 15-minute intervals. According to the results, montelukast shows a neutralizing effect at 12 micromolar and is not cytotoxic at this concentration.

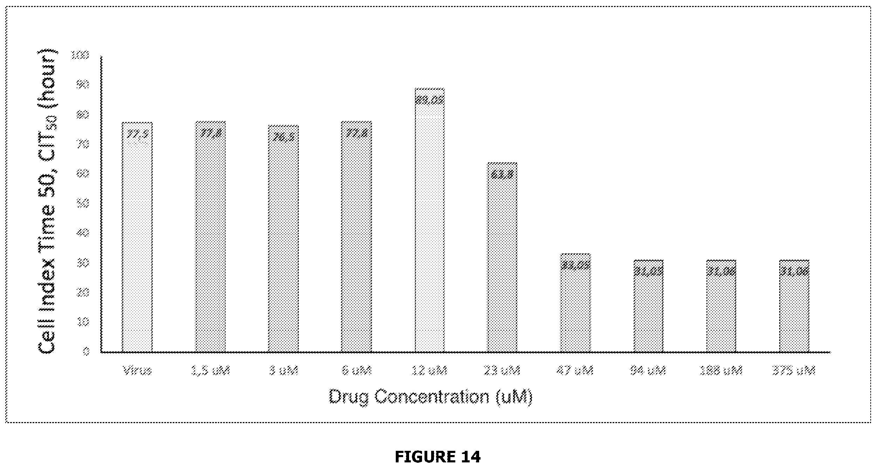

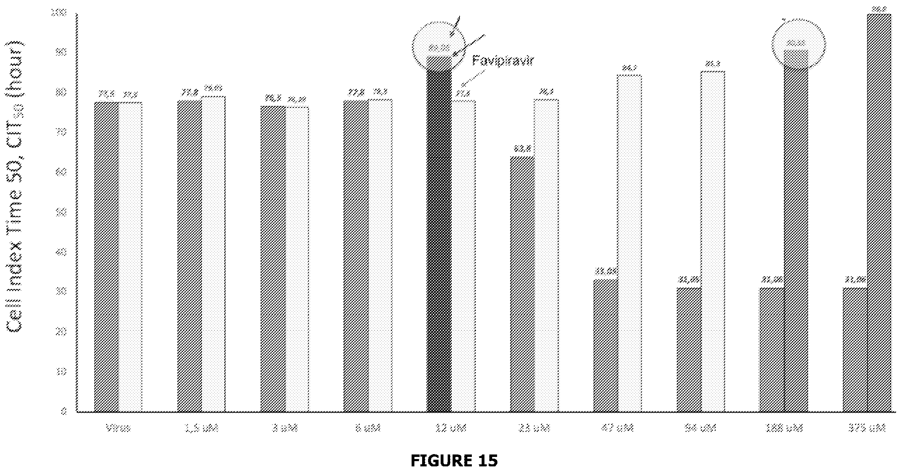

CIT50 (Cell Index Time 50), calculated from the data obtained as a result of the analyses, is the time required for the cell index to decrease by 50% after virus infection. While cytotoxicity of the molecule was not observed at a concentration of 12 micromolar, the CIT 50 value was delayed for approximately 12 hours. (Figure 14). When the same experiment was performed for Favipiravir, one of the antivirals used in the treatment of COVID-19, it was observed that in order for Favipiravir to have the same effect, it had to be used at a concentration 16 times higher than Montelukast. (Figure 15).

When evaluated together with the pseudovirus neutralization test results, it is seen that the molecule can be used at concentrations of 10 to 25 µM.

When all these results are evaluated, it has shown that the effect of Montelukast on SARS-CoV-2 occurs through dual inhibition (main protease and Spike/ACE-2) and its potential to be an effective agent in neutralizing the virus has been confirmed by *in silico* and *in vitro* experiments.

Table 3 shows chemical forms of Montelukast derivatives, IUPAC nomenclature Spike/ACE2 and Mpro binding energies.

| | IUPAC name | Molecule name | Spike /ACE-2 doking score (kkal/mol) | Mpro doking score (kkal/mol) |
|---|---|---|---|---|
| 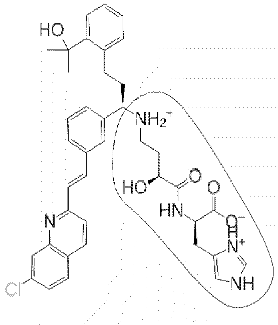 | 4-[(2R)-2-carboxylato-2-[(2S)-4- {[(1R)-1-{3-[(1E)-2-(7-chloroquinoline-2)-yl) ethyl]phenyl}-3-[2-(2-hydroxypropan-2-yl)phenyl] propyl]azaniumyl}-2-hydroxybutanamido]eth yl] -1H-imidazole-3-ium | MG-MD19 | -8.38 | -9.38 |
| 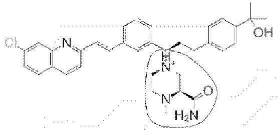 | (1R,3R)-3-carbamoyl-1-[(1R)-1-{3-[(1E)-2-(7-chloroquinoline-2-yl) ethenyl]phenyl}-3-[4-(2-hydroxypropan-2-yl) phenyl]propyl]-4-methylpiperazine-1-ium | MG-MD10 | -7.89 | -8.86 |
| 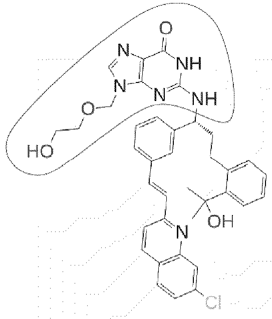 | 2-{[(1R)-1-{3-[(1E)-2-(7-chloroquinoline-2-yl)ethenyl]phenyl}-3-[2-(2-hydroxypropan-2-yl)phenyl] propyl]amino}-9-[(2-hydroxyethoxy)methyl] -6,9-dihydro-1H-purine-6-one | MG-MD13 | -7.52 | -10.30 |
| 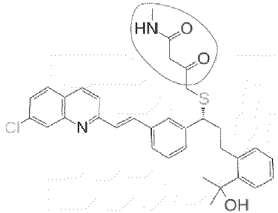 | 4-{[(1R)-1-{3-[(1E)-2-(7-chloroquinoline-2-yl)ethyl]phenyl}-3- [2-(2-hydroxypropan-2-yl)phenyl]propyl]sulfany l}-N-methyl-3-oxobutanamide | MG-MD14 | -7.424 | -9.47 |

## Claims

1. Montelukast derivative for dual inhibition of Main Protease and Spike/ACE-2 proteins in SARS-CoV-2 virus; wherein the montelukast derivative is
- 4-[(2R)-2-carboxylato-2-[(2S)-4-{[(1R)-1-{3-[(1E)-2-(7-chloroquinoline-2)-yl)ethyl]phenyl}-3-[2-(2-hydroxypropan-2-yl)phenyl]propyl]azaniumyl}-2-hydroxybutanamido]ethyl]-1H-imidazole-3-ium, or
- (1R,3R)-3-carbamoyl-1-[(1R)-1-{3-[(1E)-2-(7-chloroquinoline-2-yl)ethenyl]phenyl}-3-[4-(2-hydroxypropan-2-yl)phenyl]propyl]-4-methylpiperazine-1-ium, or
- 2-{[(1R)-1-{3-[(1E)-2-(7-chloroquinoline-2-yl)ethenyl]phenyl}-3-[2-(2-hydroxypropan-2-yl)phenyl]propyl]amino}-9-[(2-hydroxyethoxy)methyl]-6,9-dihydro-1H-purine-6-one, or
- 4-{[(1R)-1-{3-[(1E)-2-(7-chloroquinoline-2-yl)ethyl]phenyl}-3-[2-(2-hydroxypropan-2-yl)phenyl]propyl]sulfanyl}-N-methyl-3-oxobutanamide.

2. Montelukast derivative according to claim 1 for use in the prevention and treatment of COVID-19 by inhibiting Main Protease and Spike/ACE-2 proteins in SARS-CoV-2 virus.

3. Montelukast derivative according to claim 1 or montelukast derivative for use according to claim 2, wherein montelukast derivative inhibits the Mpro protein by 74% at 100 micromolar.

4. Montelukast derivative according to claims 1 or 2 or montelukast derivative for use according to claims 2 or 3, wherein the inhibition of spike/ACE-2 interface interaction of montelukast derivative is 54.04 micromolar.

5. A pharmaceutical formulation for dual inhibition of Main Protease and Spike/ACE-2 proteins in SARS-CoV-2 virus, comprising montelukast derivative and at least one pharmaceutically acceptable excipient; wherein montelukast derivative is
- 4-[(2R)-2-carboxylato-2-[(2S)-4-{[(1R)-1-{3-[(1E)-2-(7-chloroquinoline-2)-yl)ethyl]phenyl}-3-[2-(2-hydroxypropan-2-yl)phenyl]propyl]azaniumyl}-2-hydroxybutanamido]ethyl]-1H-imidazole-3-ium, or
- (1R,3R)-3-carbamoyl-1-[(1R)-1-{3-[(1E)-2-(7-chloroquinoline-2-yl)ethenyl]phenyl}-3-[4-(2-hydroxypropan-2-yl)phenyl]propyl]-4-methylpiperazine-1-ium, or
- 2-{[(1R)-1-{3-[(1E)-2-(7-chloroquinoline-2-yl)ethenyl]phenyl}-3-[2-(2-hydroxypropan-2-yl)phenyl]propyl]amino}-9-[(2-hydroxyethoxy)methyl]-6,9-dihydro-1H-purine-6-one, or
- 4-{[(1R)-1-{3-[(1E)-2-(7-chloroquinoline-2-yl)ethyl]phenyl}-3-[2-(2-hydroxypropan-2-yl)phenyl]propyl]sulfanyl}-N-methyl-3-oxobutanamide.

6. The pharmaceutical formulation according to claim 5, wherein said pharmaceutical formulation comprises an additional active ingredient which is favipiravir, arbidol, camostat mesylate, ritonavir, lopinavir, GC-376, or a combination thereof.

## Patentansprüche

1. Montelukast-Derivat zur dualen Hemmung von Hauptprotease- und Spike/ACE-2-Proteinen im SARS-CoV-2-Virus, wobei das Montelukast-Derivat
- 4-[(2R)-2-Carboxylato-2-[(2S)-4-{[(1R)-1-{3-[(1E)-2-(7-chlorchinolin-2-yl)ethyl]phenyl}-3-[2-(2-hydroxypropan-2-yl)phenyl]propyl]azaniumyl}-2-hydroxybutanamido]ethyl]-1H-imidazol-3-ium, oder
- (1R,3R)-3-Carbamoyl-1-[(1R)-1-{3-[(1E)-2-(7-chlorchinolin-2-yl)ethenyl]phenyl}-3-[4-(2-hydroxypropan-2-yl)phenyl]propyl]-4-methylpiperazin-1-ium, oder
- 2-{[(1R)-1-{3-[(1E)-2-(7-chlorchinolin-2-yl)ethenyl]phenyl}-3-[2-(2-hydroxypropan-2-yl)phenyl]propyl]amino}-9-[(2-hydroxyethoxy)methyl]-6,9-dihydro-1H-purin-6-on, oder
- 4-{[(1R)-1-{3-[(1E)-2-(7-chlorchinolin-2-yl)ethyl]phenyl}-3-[2-(2-hydroxypropan-2-yl)phenyl]propyl]sulfanyl}-N-methyl-3-oxobutanamid ist.

2. Montelukast-Derivat nach Anspruch 1 zur Verwendung bei der Vorbeugung und Behandlung von COVID-19 durch Hemmung von Hauptprotease- und Spike/ACE-2-Proteinen im SARS-CoV-2-Virus.

3. Montelukast-Derivat nach Anspruch 1 oder Montelukast-Derivat zur Verwendung nach Anspruch 2, wobei das Montelukast-Derivat das Mpro-Protein bei 100 Mikromolar zu 74 % hemmt.

4. Montelukast-Derivat nach Anspruch 1 oder 2 oder Montelukast-Derivat zur Verwendung nach Anspruch 2 oder 3, wobei die Hemmung der Spike/ACE-2-Grenzflächenwechselwirkung des Montelukast-Derivats 54,04 Mikromolar beträgt.

5. Eine pharmazeutische Formulierung zur dualen Hemmung von Hauptprotease- und Spike/ACE-2-Proteinen im SARS-CoV-2-Virus, umfassend ein Montelukast-Derivat und mindestens einen pharmazeutisch annehmbaren Hilfsstoff; wobei das Montelukast-Derivat
- 4-[(2R)-2-Carboxylato-2-[(2S)-4-{[(1R)-1-{3-[(1E)-2-(7-chlorchinolin-2-yl)ethyl]phenyl}-3-[2-(2-hydroxypropan-2-yl)phenyl]propyl]azaniumyl}-2-hydroxybutanamido]ethyl]-1H-imidazol-3-ium, oder
- (1R,3R)-3-Carbamoyl-1-[(1R)-1-{3-[(1E)-2-(7-chlorchinolin-2-yl)ethenyl]phenyl}-3-[4-(2-hydroxypropan-2-yl)phenyl]propyl]-4-methylpiperazin-1-ium, oder
- 2-{[(1R)-1-{3-[(1E)-2-(7-chlorchinolin-2-yl)ethenyl]phenyl}-3-[2-(2-hydroxypropan-2-yl)phenyl]propyl]amino}-9-[(2-hydroxyethoxy)methyl]-6,9-dihydro-1H-purin-6-on, oder
- 4-{[(1R)-1-{3-[(1E)-2-(7-chlorchinolin-2-yl)ethyl]phenyl}-3-[2-(2-hydroxypropan-2-yl)phenyl]propyl]sulfanyl}-N-methyl-3-oxobutanamid ist.

6. Die pharmazeutische Formulierung nach Anspruch 5, wobei die pharmazeutische Formulierung einen zusätzlichen Wirkstoff umfasst, bei dem es sich um Favipiravir, Arbidol, Camostat-Mesylat, Ritonavir, Lopinavir, GC-376 oder eine Kombination davon handelt.

## Revendications

1. Dérivé de montélukast pour la double inhibition de la protéase principale et des protéines Spike/ACE-2 chez le virus SARS-CoV-2 ; dans lequel le dérivé de montélukast est le :
- 4-[(2R)-2-carboxylato-2-[(2S)-4-{[(1R)-1-{3-[(1E)-2-(7-chloroquinoléine-2-yl)éthyl]phényl}-3-[2-(2-hydroxypropan-2-yl)phényl]propyl]azaniumyl}-2-hydroxybutanamido]éthyl]-1H-imidazole-3-ium, ou le
- (1R,3R)-3-carbamoyl-1-[(1R)-1-{3-[(1E)-2-(7-chloroquinoléine-2-yl)éthényl]phényl}-3-[4-(2-hydroxypropan-2-yl)phényl]propyl]-4-méthylpipérazine-1-ium, ou la
- 2-{[(1R)-1-{3-[(1E)-2-(7-chloroquinoléine-2-yl)éthényl]phényl}-3-[2-(2-hydroxypropan-2-yl)phényl]propyl]amino}-9-[(2-hydroxyéthoxy)méthyl]-6,9-dihydro-1H-purine-6-one, ou le
- 4-{[(1R)-1-{3-[(1E)-2-(7-chloroquinoléine-2-yl)éthyl]phényl}-3-[2-(2-hydroxypropan-2-yl)phényl]propyl]sulfanyl}-N-méthyl-3-oxobutanamide.

2. Dérivé de montélukast selon la revendication 1 pour son utilisation dans la prévention et le traitement de la COVID-19 par inhibition de la protéase principale et des protéines Spike/ACE-2 chez le virus SARS-CoV-2.

3. Dérivé de montélukast selon la revendication 1 ou dérivé de montélukast pour son utilisation selon la revendication 2, dans lequel le dérivé de montélukast inhibe la protéine Mpro à hauteur de 74 % à 100 micromolaire.

4. Dérivé de montélukast selon les revendications 1 ou 2 ou dérivé de montélukast pour son utilisation selon les revendications 2 ou 3, dans lequel l'inhibition de l'interaction à l'interface Spike/ACE-2 du dérivé de montélukast est de 54,04 micromolaire.

5. Formulation pharmaceutique pour la double inhibition de la protéase principale et des protéines Spike/ACE-2 chez le virus SARS-CoV-2, comprenant un dérivé de montélukast et au moins un excipient pharmaceutiquement acceptable ; dans laquelle le dérivé de montélukast est le :
- 4-[(2R)-2-carboxylato-2-[(2S)-4-{[(1R)-1-{3-[(1E)-2-(7-chloroquinoléine-2-yl)éthyl]phényl}-3-[2-(2-hydroxypropan-2-yl)phényl]propyl]azaniumyl}-2-hydroxybutanamido]éthyl]-1H-imidazole-3-ium, ou le
- (1R,3R)-3-carbamoyl-1-[(1R)-1-{3-[(1E)-2-(7-chloroquinoléine-2-yl)éthényl]phényl}-3-[4-(2-hydroxypropan-2-yl)phényl]propyl]-4-méthylpipérazine-1-ium, ou la
- 2-{[(1R)-1-{3-[(1E)-2-(7-chloroquinoléine-2-yl)éthényl]phényl}-3-[2-(2-hydroxypropan-2-yl)phényl]propyl]amino}-9-[(2-hydroxyéthoxy)méthyl]-6,9-dihydro-1H-purine-6-one, ou le
- 4-{[(1R)-1-{3-[(1E)-2-(7-chloroquinoléine-2-yl)éthyl]phényl}-3-[2-(2-hydroxypropan-2-yl)phényl]propyl]sulfanyl}-N-méthyl-3-oxobutanamide.

6. Formulation pharmaceutique selon la revendication 5, dans laquelle ladite formulation pharmaceutique comprend un principe actif supplémentaire qui est le favipiravir, l'arbidol, le mésylate de camostat, le ritonavir, le lopinavir, le GC-376, ou une combinaison de ceux-ci.
